# EUROPEAN PATENT APPLICATION

(11) **EP 3 904 356 A1**
(43) Date of publication of application: **03.11.2021**
(21) Application number: 19905962.7
(22) Date of filing: 24.12.2019
(51) Int. Cl.: C07D 487/04, A61K 31/519, A61K 9/20, A61P 35/00

(54) **TREATMENT USE OF PYRROLOPYRIMIDINE COMPOUND, AND SOLID PHARMACEUTICAL COMPOSITION OF PYRROLOPYRIMIDINE COMPOUND**

(30) Priority: 24.12.2018 CN 201811581815; 26.07.2019 CN 201910679429
(71) Applicant: Chia Tai Tianqing Pharmaceutical Group Co., Ltd., Lianyungang, Jiangsu 222062 (CN); Lianyungang Runzhong Pharmaceutical Co., Ltd., Lianyungang, Jiangsu 222069 (CN); Shouyao Holdings (Beijing) Co., Ltd., Beijing 100176 (CN)
(72) Inventor: WANG, Dong, Lianyungang City Jiangsu 222062 (CN); LI, Qingxia, Lianyungang City Jiangsu 222062 (CN); DAI, Jun, Lianyungang City Jiangsu 222062 (CN); LI, Chen, Lianyungang City Jiangsu 222062 (CN); JIANG, Zhulian, Lianyungang City Jiangsu 222062 (CN); SUN, Yanqing, Lianyungang City Jiangsu 222062 (CN); CHEN, Jingjing, Lianyungang City Jiangsu 222062 (CN); JIN, Lingling, Lianyungang City Jiangsu 222062 (CN); LIU, Jundong, Lianyungang City Jiangsu 222062 (CN); LI, Qide, Lianyungang City Jiangsu 222062 (CN)
(74) Representative: Grünecker Patent- und Rechtsanwälte PartG mbB
(86) International application number: PCT/CN2019/127837
(87) International publication number: WO 2020/135401

(57) **Abstract**

A treatment use of a pyrrolopyrimidine compound, and a solid pharmaceutical composition of a pyrrolopyrimidine compound. In particular, the present invention relates to a pyrrolopyrimidine compound or a pharmaceutical composition thereof for treating myeloproliferative neoplasms, and a method therefor or a use thereof.

## Description

### CROSS-REFERENCE TO RELATED APPLICATIONS

The present application claims the benefit and priority to the Chinese Patent Application No. 201811581815.8 filed with the China National Intellectual Property Administration on Dec. 24, 2018 and the Chinese Patent Application No. 201910679429.0 filed with the China National Intellectual Property Administration on Jul. 26, 2019, which are incorporated herein by reference in their entirety.

### TECHNICAL FIELD

The present application relates to the field of medicinal chemistry, and particularly, to therapeutic use and a solid pharmaceutical composition of a pyrrolopyrimidine compound.

### BACKGROUND

Janus kinase (JAK), which exists in cells and transmits cytokine stimulation signals through the JAK-STAT pathway, is a group of non-receptor tyrosine kinases (nRTKs). The JAK-STAT pathway transmits extracellular chemical signals through the cell membrane to the gene promoter of DNA in the nucleus, which ultimately causes changes in DNA transcription and activity. The JAK-STAT pathway consists of three major components: 1) a receptor; 2) the JAK; and 3) a signal transducer and activator of transcription (STAT) protein. The receptor can be activated by interferons, interleukins, growth factors or other chemical messengers, leading to the autophosphorylation of JAK; the STAT protein binds to the phosphorylated receptor, such that the STAT protein is phosphorylated by JAK; then the phosphorylated STAT protein is separated from the receptor, dimerized and translocated into the nucleus to bind to the specific sites on DNA and alter the transcription (Scott, M. J., C. J. Godshall, et al., (2002) "Jaks, STATs, Cytokines, and Sepsis", Clin Diagn Lab Immunol 9(6):1153-9).

The JAK family plays a role in cell proliferation and functional cytokine-dependent regulation of immune responses. Currently, there are four known mammalian JAK family members: JAK1, JAK2, JAK3 and TYK2 (tyrosine kinase 2). The size of JAK proteins ranges from 120-140 kDa. A JAK protein contains 7 conserved JAK homology (JH) domains, one of which is a functional catalytic kinase domain, and another of which is a pseudokinase domain that effectively exerts a regulatory function and/or acts as a docking site for STAT proteins (Scott, Godshall, et al., 2002, supra).

Myeloproliferative neoplasms (MPNs) are clonal hematopoietic stem cell diseases characterized by the excessive proliferation of relatively mature cells of one or more lines of bone marrow. MPNs mainly include polycythemia vera (PV), essential thrombocythemia (ET) and primary myelofibrosis (PMF).

### BRIEF SUMMARY

The present application provides a compound of formula I, a stereoisomer or a pharmaceutically acceptable salt thereof for use in treating myeloproliferative neoplasm: 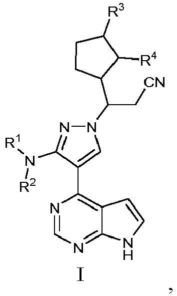 wherein, R¹ and R² are each independently selected from the group consisting of H, C₁₋₆ alkyl, C₁₋₆ alkylacyl and C₁₋₆ alkylsulfonyl; R³ and R⁴ are each independently selected from the group consisting of H, hydroxyl and oxo.

In another aspect, the present application provides a pharmaceutical composition for treating myeloproliferative neoplasm, comprising the compound of formula **I,** the stereoisomer or the pharmaceutically acceptable salt thereof as described above.

In another aspect, the present application provides a method for treating myeloproliferative neoplasm, comprising administering to a subject an effective amount of the compound of formula I, the stereoisomer or the pharmaceutically acceptable salt thereof, or the pharmaceutical composition thereof as described above.

In another aspect, the present application provides use of the compound of formula **I,** the stereoisomer or the pharmaceutically acceptable salt thereof, or the pharmaceutical composition thereof as described above in preparing a medicament for treating myeloproliferative neoplasm.

In another aspect, the present application provides use of the compound of formula **I,** the stereoisomer or the pharmaceutically acceptable salt thereof, or the pharmaceutical composition thereof as described above in treating myeloproliferative neoplasm.

In yet another aspect, the present application provides a solid pharmaceutical composition, comprising a compound of formula **I,** or a stereoisomer or a pharmaceutically acceptable salt thereof, and a diluent, a binder, a wetting agent, and a disintegrant.

In yet another aspect, the present application provides the solid pharmaceutical composition as described above for use in treating a disease mediated by JAK

In yet another aspect, the present application provides use of the solid pharmaceutical composition as described above in preparing a medicament for treating a disease mediated by JAK.

In yet another aspect, the present application provides a method for treating a disease mediated by JAK, comprising administering to a subject an effective amount of the solid pharmaceutical composition as described above.

In another aspect, the present application provides use of the solid pharmaceutical composition as described above in treating a disease mediated by JAK

### SUMMARY

The present application provides a compound of formula **I,** a stereoisomer or a pharmaceutically acceptable salt thereof for use in treating myeloproliferative neoplasm: 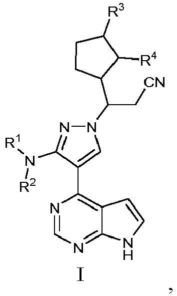 wherein, R¹ and R² are each independently selected from the group consisting of H, C₁₋₆ alkyl, C₁₋₆ alkylacyl and C₁₋₆ alkylsulfonyl; R³ and R⁴ are each independently selected from the group consisting of H, hydroxyl and oxo.

In another aspect, the present application provides a pharmaceutical composition for treating myeloproliferative neoplasm, comprising the compound of formula **I,** the stereoisomer or the pharmaceutically acceptable salt thereof as described above.

In another aspect, the present application provides a method for treating myeloproliferative neoplasm, comprising administering to a subject an effective amount of the compound of formula **I,** the stereoisomer or the pharmaceutically acceptable salt thereof, or the pharmaceutical composition thereof as described above.

In another aspect, the present application provides use of the compound of formula **I,** the stereoisomer or the pharmaceutically acceptable salt thereof, or the pharmaceutical composition thereof as described above in preparing a medicament for treating myeloproliferative neoplasm.

In another aspect, the present application provides use of the compound of formula **I,** the stereoisomer or the pharmaceutically acceptable salt thereof, or the pharmaceutical composition thereof as described above in treating myeloproliferative neoplasm.

In some embodiments of the present application, the pharmaceutical composition comprises the compound of formula **I,** or the stereoisomer or the pharmaceutically acceptable salt thereof as described above.

In some embodiments of the present application, R¹ and R² are each independently selected from the group consisting of H, methyl, ethyl, propyl, butyl, pentyl, hexyl, formyl, acetyl, propanoyl, butyryl, valeryl, hexanoyl, methylsulfonyl, ethylsulfonyl, propylsulfonyl, butylsulfonyl, pentylsulfonyl and hexylsulfonyl.

In some embodiments of the present application, R¹ is H, and R² is selected from the group consisting of methyl, ethyl, propyl, butyl, pentyl, hexyl, formyl, acetyl, propanoyl, butyryl, valeryl, hexanoyl, methylsulfonyl, ethylsulfonyl, propylsulfonyl, butylsulfonyl, pentylsulfonyl and hexylsulfonyl.

In some embodiments of the present application, the compound of formula **I** has a configuration shown in the following formulas:

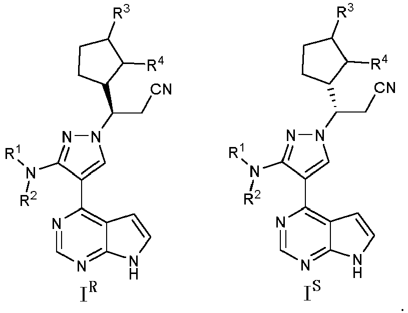

In some embodiments of the present application, the compound of formula **I,** or the stereoisomer or the pharmaceutically acceptable salt thereof is selected from the group consisting of:

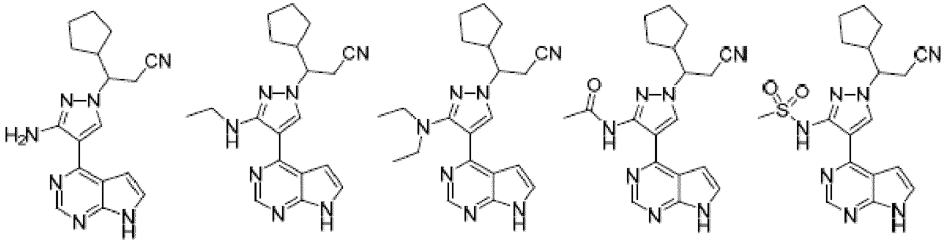

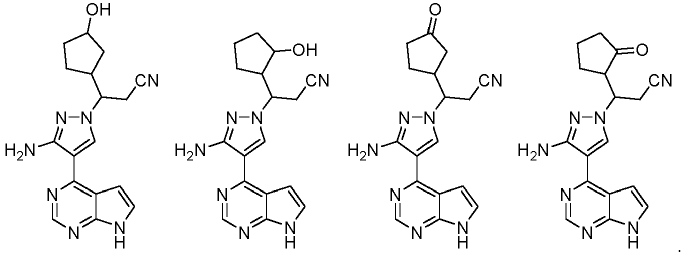

In some embodiments of the present application, the compound of formula **I,** or the stereoisomer or the pharmaceutically acceptable salt thereof is preferably selected from the group consisting of:

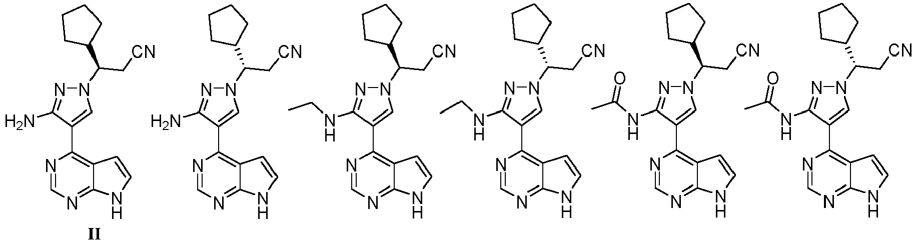

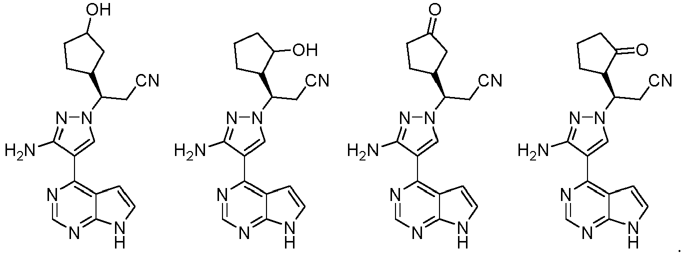

In some embodiments of the present application, a hydrochloride salt of the compound of formula **I** is preferred. In some embodiments of the present application, a monohydrochloride salt of the compound of formula **I** is preferred. In some embodiments of the present application, a crystalline monohydrochloride salt of the compound of formula **I** is preferred.

In some embodiments of the present application, a free base of the compound of formula **I** is preferred. In some embodiments of the present application, a crystalline free base of the compound of formula **I** is preferred.

In some embodiments of the present application, the compound of formula **I** is a compound of formula **II:**

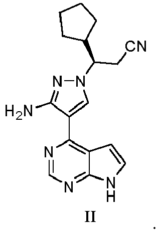

In some embodiments of the present application, a compound of formula **II** or a pharmaceutically acceptable salt thereof for use in treating polycythemia vera is provided.

In some embodiments of the present application, a compound of formula **II** or a pharmaceutically acceptable salt thereof for use in treating thrombocythemia is provided.

In some embodiments of the present application, a compound of formula **II** or a pharmaceutically acceptable salt thereof for use in treating myelofibrosis is provided.

In some embodiments of the present application, a pharmaceutical composition for use in treating polycythemia vera is provided, comprising the compound of formula **II** or the pharmaceutically acceptable salt thereof as described above.

In some embodiments of the present application, a pharmaceutical composition for use in treating thrombocythemia is provided, comprising the compound of formula **II** or the pharmaceutically acceptable salt thereof as described above.

In some embodiments of the present application, a pharmaceutical composition for use in treating myelofibrosis is provided, comprising the compound of formula **II** or the pharmaceutically acceptable salt thereof as described above.

In some embodiments of the present application, a method for treating polycythemia vera is provided, comprising administering to a subject an effective amount of the compound of formula **II** or the pharmaceutically acceptable salt thereof, or the pharmaceutical composition thereof as described above.

In some embodiments of the present application, a method for treating thrombocythemia is provided, comprising administering to a subject an effective amount of the compound of formula **II** or the pharmaceutically acceptable salt thereof, or the pharmaceutical composition thereof as described above.

In some embodiments of the present application, a method for treating myelofibrosis is provided, comprising administering to a subject an effective amount of the compound of formula **II** or the pharmaceutically acceptable salt thereof, or the pharmaceutical composition thereof as described above.

In some embodiments of the present application, use of the compound of formula **II** or the pharmaceutically acceptable salt thereof, or the pharmaceutical composition thereof in preparing a medicament for treating polycythemia vera is provided.

In some embodiments of the present application, use of the compound of formula **II** or the pharmaceutically acceptable salt thereof, or the pharmaceutical composition thereof in preparing a medicament for treating thrombocythemia is provided.

In some embodiments of the present application, use of the compound of formula **II** or the pharmaceutically acceptable salt thereof, or the pharmaceutical composition thereof in preparing a medicament for treating myelofibrosis is provided.

In some embodiments of the present application, use of the compound of formula **II** or the pharmaceutically acceptable salt thereof, or the pharmaceutical composition thereof in treating polycythemia vera is provided.

In some embodiments of the present application, use of the compound of formula **II** or the pharmaceutically acceptable salt thereof, or the pharmaceutical composition thereof in treating thrombocythemia is provided.

In some embodiments of the present application, use of the compound of formula **II** or the pharmaceutically acceptable salt thereof, or the pharmaceutical composition thereof in treating myelofibrosis is provided.

The pharmaceutical composition disclosed herein can be prepared by combining the compound disclosed herein with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, and aerosol. The pharmaceutical composition disclosed herein can be manufactured by methods well known in the art, such as conventional mixing, dissolving, granulating, dragee-making, levigating, emulsifying, and lyophilizing. Suitable excipients include, but are not limited to: binders, diluents, wetting agents, disintegrants, lubricants, glidants, sweeteners or flavoring agents, and the like.

In some embodiments of the present application, the pharmaceutical composition is a formulation suitable for oral administration, including tablets, capsules, powders, granules, dripping pills, pastes, pulvis, and the like, preferably tablets and capsules. The oral formulation can be prepared by a conventional method using a pharmaceutically acceptable carrier well known in the art. The pharmaceutically acceptable carrier includes diluents, binders, wetting agents, disintegrants, lubricants, and the like. The diluents include microcrystalline cellulose, mannitol, lactose, sucrose, starch, pregelatinized starch, dextrin, etc., or a mixture thereof; the binders include hydroxypropyl methylcellulose, carboxymethyl cellulose, sodium carboxymethyl cellulose, ethyl cellulose, methyl cellulose, hydroxypropyl cellulose, low-substituted hydroxypropyl cellulose, gelatin, polyvinylpyrrolidone, starch, sucrose, glucose, gelatin, etc., or a mixture thereof; the wetting agents include magnesium stearate, talc, polyethylene glycol, sodium dodecyl sulfate, silica gel micropowder, talc, etc., or a mixture thereof; the disintegrants include sodium carboxymethyl starch, dry starch, microcrystalline cellulose, hydroxyethyl methyl cellulose, sodium carboxymethyl cellulose, calcium carboxymethyl cellulose, croscarmellose sodium, low-substituted hydroxypropyl methylcellulose, crospovidone, etc., or a mixture thereof; and the lubricants include magnesium stearate, colloidal silicon dioxide, talc, polyethylene glycol, stearic acid, sodium stearoyl fumarate, etc., or a mixture thereof. The pharmaceutical excipients also include coloring agents, sweeteners, coating agents, and the like.

In some embodiments of the present application, the pharmaceutical composition is in a unit dosage form. In some embodiments, the pharmaceutical composition comprises 1 mg to 50 mg of the compound, or the stereoisomer or the pharmaceutically acceptable salt thereof disclosed herein. In some embodiments, the pharmaceutical composition comprises 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg or 50 mg, or any range defined by endpoints of any of the foregoing values or any value in the range of the compound, or the stereoisomer or the pharmaceutically acceptable salt thereof disclosed herein, for example, 2 mg to 50 mg, 10 mg to 40 mg, 5 mg to 30 mg, 5 mg to 20 mg, etc.

### Solid Pharmaceutical Composition

The present application provides a solid pharmaceutical composition, comprising a compound of formula **I** or a compound of formula **II,** or a stereoisomer or a pharmaceutically acceptable salt thereof, and a diluent, a binder, a wetting agent and a disintegrant.

In some embodiments of the application, the solid pharmaceutical composition comprises a compound of formula **I** or a compound of formula **II,** and a diluent, a binder, a wetting agent and a disintegrant.

In some embodiments of the present application, the solid pharmaceutical composition further comprises a lubricant. In some embodiments of the present application, the amount of the compound of formula **I** or the compound of formula **II** is selected from 1-30% wt, preferably 1-25% wt, 1-20% wt, 2-20% wt, 2-15% wt, 2-10% wt, 3-10% wt, or 2-8% wt, more preferably 3-8% wt, further more preferably 3.5-6% wt; or in some embodiments, the amount of the compound of formula **I** or compound of formula **II** is selected from the group consisting of 1% wt, 1.2% wt, 1.4% wt, 1.6% wt, 1.8% wt, 2% wt, 2.2% wt, 2.4% wt, 2.6% wt, 2.8% wt, 3% wt, 3.2% wt, 3.4% wt, 3.6% wt, 3.8% wt, 4% wt, 4.2% wt, 4.4% wt, 4.6% wt, 4.8% wt, 5% wt, 5.2% wt, 5.4% wt, 5.6% wt, 5.8% wt, 6% wt, 6.2% wt, 6.4% wt, 6.6% wt, 6.8% wt, 7% wt, 7.2% wt, 7.4% wt, 7.6% wt, 7.8% wt, 8% wt, 8.2% wt, 8.4% wt, 8.6% wt, 8.8% wt, 9% wt, 9.2% wt, 9.4% wt, 9.6% wt, 9.8% wt, 10% wt, 10.5% wt, 11% wt, 11.5% wt, 12% wt, 12.5% wt, 13% wt, 13.5% wt, 14% wt, 14.5% wt, 15% wt, 15.5% wt, 16% wt, 16.5% wt, 17% wt, 17.5% wt, 18% wt, 18.5% wt, 19% wt, 19.5% wt, 20% wt, 21% wt, 22% wt, 23% wt, 24% wt, 25% wt, 26% wt, 27% wt, 28% wt, 29% wt and 30% wt, or any range defined by endpoints of any of the foregoing values, or any value in the range.

In some embodiments of the present application, the diluent is selected from the group consisting of microcrystalline cellulose, mannitol, lactose, sucrose, starch, pregelatinized starch and dextrin, or a mixture thereof; preferably, microcrystalline cellulose, mannitol, lactose and pregelatinized starch, or a mixture thereof; more preferably, microcrystalline cellulose and mannitol, or a mixture thereof. In some embodiments of the present application, the amount of the diluent is selected from 50-95% wt, preferably 60-95% wt, 65-95% wt, 70-95% wt, 75-95% wt, 80-95% wt, 80-90% wt or 85-95% wt, more preferably 85-90% wt; or in some embodiments, the amount of the diluent is selected from the group consisting of 55% wt, 60% wt, 65% wt, 70% wt, 75% wt, 80% wt, 85% wt, 90% wt and 95% wt, or any range defined by endpoints of any of the foregoing values, or any value in the range. In some embodiments of the present application, the binder is selected from the group consisting of hydroxypropyl methylcellulose (HPMC), carboxymethyl cellulose (CMC), sodium carboxymethyl cellulose (CMC-Na), ethyl cellulose (EC), methyl cellulose (MC), hydroxypropyl cellulose (HPC), low-substituted hydroxypropyl cellulose (L-HPC), gelatin, polyvinylpyrrolidone (PVP), partially hydrolyzed starch, starch, pregelatinized starch, sucrose, glucose, gelatin, polyethylene glycol (PEG) and polyvinyl alcohol, or a mixture thereof; preferably hydroxypropyl methylcellulose (HPMC), carboxymethyl cellulose (CMC), sodium carboxymethyl cellulose (CMC-Na), ethyl cellulose (EC), methyl cellulose (MC), hydroxypropyl cellulose (HPC), low-substituted hydroxypropyl cellulose (L-HPC) and polyvinylpyrrolidone, or a mixture thereof; more preferably hydroxypropyl cellulose and polyvinylpyrrolidone, or a mixture thereof.

In some embodiments of the present application, the amount of the binder is selected from 1.0-10% wt, preferably 1.0-8.0% wt, 1.0-6.0% wt, or 1.0-5.0% wt, more preferably 2.0-4.0% wt; or in some embodiments, the amount of the binder is selected from the group consisting of 1.0% wt, 1.5% wt, 2.0% wt, 2.5% wt, 3.0% wt, 3.5% wt, 4.0% wt, 4.5% wt, 5.0% wt, 5.5% wt, 6.0% wt, 6.5% wt, 7.0% wt, 7.5% wt, 8.0% wt, 8.5% wt, 9.0% wt, 9.5% wt and 10% wt, or any range defined by endpoints of any of the foregoing values or any value in the range.

In some embodiments of the present application, the wetting agent is selected from the group consisting of sodium dodecylbenzene sulfonate, magnesium dodecylbenzene sulfonate, sodium tetradecylbenzene sulfonate, sodium hexadecylbenzene sulfonate, sodium octadecylbenzene sulfonate, sodium dodecyl sulfonate, magnesium dodecyl sulfonate, sodium tetradecyl sulfonate, sodium hexadecyl sulfonate, sodium octadecyl sulfonate, sodium dodecyl sulfate, magnesium dodecyl sulfate, sodium tetradecyl sulfate, sodium hexadecyl sulfate, sodium octadecyl sulfate, sodium lauroyl sarcosine, sodium lactate, sodium palmitate, lauric isopropanolamide, lauric diethanolamide, tetradecyl lactate, hexadecyl lactate, polysorbate 20, polysorbate 40, polysorbate 60, polysorbate 65, polysorbate 80, polysorbate 85, polyoxyethylene lauryl ether, polyoxyethylene cetyl ether, polyoxyethylene sorbitol tetraoleyl ether, polyoxyethylene stearate, polyoxyethylene castor oil and polyoxyethylene hydrogenated castor oil, or a mixture thereof; preferably, sodium dodecyl sulfonate, magnesium dodecyl sulfonate, sodium tetradecyl sulfonate, sodium dodecyl sulfate, magnesium dodecyl sulfate, sodium tetradecyl sulfate, sodium hexadecyl sulfate, sodium octadecyl sulfate and sodium lauroyl sarcosine, or a mixture thereof; more preferably, sodium dodecyl sulfate and magnesium dodecyl sulfate, or a mixture thereof.

In some embodiments of the present application, the amount of the wetting agent is selected from 0.01-5.0% wt, preferably 0.01-4.0% wt, 0.01-3.0% wt, 0.02-2.5% wt, 0.02-2.0% wt, more preferably 0.03-2.0% wt, further more preferably 0.05-1.0% wt, still more preferably 0.1-0.5% wt; or in some embodiments, the amount of the lubricant is selected from the group consisting of 0.01% wt, 0.02% wt, 0.03% wt, 0.04% wt, 0.05% wt, 0.06% wt, 0.07% wt, 0.08% wt, 0.09% wt, 0.1% wt, 0.2% wt, 0.3% wt, 0.4% wt, 0.5% wt, 0.6% wt, 0.7% wt, 0.8% wt, 0.9% wt, 1.0% wt, 1.1% wt, 1.2% wt, 1.3% wt, 1.4% wt, 1.5% wt, 1.6% wt, 1.7% wt, 1.8% wt, 1.9% wt, 2.0% wt, 2.1% wt, 2.2% wt, 2.3% wt, 2.4% wt, 2.5% wt, 2.6% wt, 2.7% wt, 2.8% wt, 2.9% wt, 3.0% wt, 3.2% wt, 3.4% wt, 3.6% wt, 3.8% wt, 4.0% wt, 4.2% wt, 4.4% wt, 4.6% wt, 4.8% wt and 5.0% wt, or any range defined by endpoints of any of the foregoing values, or any value in the range.

In some embodiments of the present application, the disintegrant is selected from the group consisting of sodium carboxymethyl starch, dry starch, microcrystalline cellulose, hydroxyethyl methyl cellulose, sodium carboxymethyl cellulose, calcium carboxymethyl cellulose, croscarmellose sodium, low-substituted hydroxypropyl methyl cellulose or crospovidone, sodium dodecyl sulfate and magnesium dodecyl sulfate, or a mixture thereof; preferably sodium carboxymethyl starch and croscarmellose sodium, or a mixture thereof.

In some embodiments of the present application, the amount of the disintegrant is selected from 1.0-7.0% wt, preferably 1.0-6.5% wt, 1.0-6.5% wt, 1.0-6.0% wt, 1.5-5.5% wt, 1.5-5.0% wt or 1.5-4.5% wt, more preferably 2.0-4.0% wt; or in some embodiments, the amount of the disintegrant is selected from the group consisting of 1.0% wt, 1.5% wt, 2.0% wt, 2.5% wt, 3.0% wt, 3.5% wt, 4.0% wt, 4.5% wt, 5.0% wt, 5.5% wt, 6.0% wt, 6.5% wt and 7.0% wt, or any range defined by endpoints of any of the foregoing values, or any value in the range.

In some embodiments of the present application, the lubricant is selected from the group consisting of magnesium stearate, colloidal silicon dioxide, talc, polyethylene glycol 4000, polyethylene glycol 6000, stearic acid, sodium stearyl fumarate and sodium dodecyl sulfate, or a mixture thereof, preferably magnesium stearate and colloidal silicon dioxide, or a mixture thereof.

In some embodiments of the present application, the amount of the lubricant is selected from 0.1-3% wt, preferably 0.2-2.5% wt, 0.3-2.0% wt, or 0.4-1.5% wt, more preferably 0.5-1% wt; or in some embodiments, the amount of the lubricant is selected from the group consisting of 0.1% wt, 0.2% wt, 0.3% wt, 0.4% wt, 0.5% wt, 0.6% wt, 0.7% wt, 0.8% wt, 0.9% wt, 1.0% wt, 1.1% wt, 1.2% wt, 1.3% wt, 1.4% wt, 1.5% wt, 1.6% wt, 1.7% wt, 1.8% wt, 1.9% wt, 2.0% wt, 2.1% wt, 2.2% wt, 2.3% wt, 2.4% wt, 2.5% wt, 2.6% wt, 2.7% wt, 2.8% wt, 2.9% wt and 3.0% wt, or any range defined by endpoints of any of the foregoing values, or any value in the range.

In some embodiments of the present application, the solid pharmaceutical composition comprises 50-95% wt of microcrystalline cellulose, mannitol, lactose or pregelatinized starch, or a mixture thereof; preferably 60-95% wt, 65-95% wt, 70-95% wt, 75-95% wt, 80-95% wt, 80-90% wt or 85-95% wt of microcrystalline cellulose, mannitol, lactose or pregelatinized starch, or a mixture thereof; more preferably 85-90% wt of microcrystalline cellulose, mannitol, lactose, and pregelatinized starch, or a mixture thereof; further more preferably 85-90% wt of microcrystalline cellulose or mannitol, or a mixture thereof. In some preferred embodiments, the diluent is a mixture of microcrystalline cellulose and mannitol, wherein the weight ratio of microcrystalline cellulose to mannitol is selected from 1:1 to 5:1, preferably 1:1 to 4:1, 1.2:1 to 3.5:1 or 1.2:1 to 3:1, more preferably 1.5:1 to 2.5:1; or the weight ratio of microcrystalline cellulose to mannitol is selected from the group consisting of 1:1, 1.2:1, 1.5:1, 1.8:1, 1.9:1, 2:1, 2.2:1, 2.5:1, 2.8:1, 3:1, 3.2:1, 3.5:1, 3.8:1, 4:1, 4.2:1, 4.5:1, 4.8:1 and 5:1.

In some embodiments of the present application, based on the amount of the solid pharmaceutical composition, the diluent in the solid pharmaceutical composition is selected from 50-70% wt of microcrystalline cellulose and 20-40% wt of mannitol, preferably 52-68% wt of microcrystalline cellulose and 22-38% wt of mannitol, 54-66% wt of microcrystalline cellulose and 24-36% wt of mannitol, 54-64% wt of microcrystalline cellulose and 24-34% wt of mannitol, or 56-62% wt of microcrystalline cellulose and 26-32% wt of mannitol, more preferably 56-60% wt of microcrystalline cellulose and 26-30% wt of mannitol, further more preferably 58.3% wt of microcrystalline cellulose and 29.4% wt of mannitol.

In some embodiments of the present application, the solid pharmaceutical composition comprises 1.0-10% wt of HPMC, CMC, CMC-Na, EC, MC, HPC, L-HPC or PVP, or a mixture thereof; preferably 1.0-8.0% wt, 1.0-6.0% wt or 1.0-5.0% wt of HPMC, CMC, CMC-Na, EC, MC, HPC, L-HPC or PVP, or a mixture thereof; more preferably 2.0-4.0% wt of HPMC, CMC, CMC-Na, EC, MC, HPC or L-HPC, or a mixture thereof; further more preferably 2.0-4.0% wt of HPC or PVP, or a mixture thereof.

In some embodiments of the present application, the solid pharmaceutical composition comprises 0.01-5.0% wt of sodium dodecyl sulfonate, magnesium dodecyl sulfonate, sodium tetradecyl sulfonate, sodium dodecyl sulfate, magnesium dodecyl sulfate, sodium tetradecyl sulfate, sodium hexadecyl sulfate, sodium octadecyl sulfate or sodium lauroyl sarcosine, or a mixture thereof; preferably 0.01-4.0% wt, 0.01-3.0% wt, 0.02-2.5% wt or 0.02-2.0% wt of sodium dodecyl sulfonate, magnesium dodecyl sulfonate, sodium tetradecyl sulfonate, sodium dodecyl sulfate, magnesium dodecyl sulfate, sodium tetradecyl sulfate, sodium hexadecyl sulfate, sodium octadecyl sulfate or sodium lauroyl sarcosine, or a mixture thereof; more preferably 0.03-2.0% wt of sodium dodecyl sulfonate, magnesium dodecyl sulfonate, sodium tetradecyl sulfonate, sodium dodecyl sulfate, magnesium dodecyl sulfate, sodium tetradecyl sulfate, sodium hexadecyl sulfate, sodium octadecyl sulfate or sodium lauroyl sarcosine, or a mixture thereof; further more preferably 0.03-2.0% wt of sodium dodecyl sulfate or magnesium dodecyl sulfate, or a mixture thereof; still more preferably 0.1-0.5% wt of sodium dodecyl sulfate or magnesium dodecyl sulfate, or a mixture thereof.

In some embodiments of the present application, the solid pharmaceutical composition comprises 1.0-7.0% wt of sodium carboxymethyl starch or croscarmellose sodium, or a mixture thereof; preferably 1.0-6.5% wt, 1.0-6.5% wt, 1.0-6.0% wt, 1.5-5.5% wt, 1.5-5.0% wt or 1.5-4.5% wt of sodium carboxymethyl starch or croscarmellose sodium, or a mixture thereof; more preferably 2.0-4.0% wt of sodium carboxymethyl starch or croscarmellose sodium, or a mixture thereof.

In some embodiments of the present application, the solid pharmaceutical composition comprises 0.1-3% wt of magnesium stearate or colloidal silicon dioxide, or a mixture thereof; preferably 0.2-2.5% wt, 0.3-2.0% wt or 0.4-1.5% wt of magnesium stearate or colloidal silicon dioxide, or a mixture thereof; more preferably 0.5-1% wt of magnesium stearate or colloidal silicon dioxide, or a mixture thereof.

In some embodiments of the present application, the solid pharmaceutical composition disclosed herein comprises:
A) 1-30% wt of the compound of formula **I** or the compound of formula **II;**
   50-95% wt of microcrystalline cellulose, mannitol, lactose or pregelatinized starch, or a mixture thereof;
   1.0-10% wt of HPMC, CMC, CMC-Na, EC, MC, HPC, L-HPC or PVP, or a mixture thereof;
   0.01-5.0% wt of sodium dodecyl sulfonate, magnesium dodecyl sulfonate, sodium tetradecyl sulfonate, sodium dodecyl sulfate, magnesium dodecyl sulfate, sodium tetradecyl sulfate, sodium hexadecyl sulfate, sodium octadecyl sulfate or sodium lauroyl sarcosine, or a mixture thereof;
   1.0-7.0% wt of sodium carboxymethyl starch or croscarmellose sodium, or a mixture thereof; and optionally, 0.1-3% wt of magnesium stearate or colloidal silicon dioxide, or a mixture thereof; or
B) 2-10% wt of the compound of formula **I** or the compound of formula **II;**
   75-95% wt of microcrystalline cellulose or mannitol, or a mixture thereof, wherein the weight ratio of microcrystalline cellulose to mannitol in the mixture is selected from 1.2:1 to 3.5:1;
   1.0-6.0% wt of hydroxypropyl cellulose or polyvinylpyrrolidone, or a mixture thereof;
   0.01-3.0% wt of sodium dodecyl sulfonate, magnesium dodecyl sulfonate, sodium tetradecyl sulfonate, sodium dodecyl sulfate, magnesium dodecyl sulfate, sodium tetradecyl sulfate, sodium hexadecyl sulfate, sodium octadecyl sulfate or sodium lauroyl sarcosine, or a mixture thereof;
   1.0-6.0% wt of sodium carboxymethyl starch or croscarmellose sodium, or a mixture thereof; and optionally, 0.3-2.0% wt of magnesium stearate or colloidal silicon dioxide, or a mixture thereof; or
C) 3-8% wt of the compound of formula **I** or the compound of formula **II;**
   85-90% wt of microcrystalline cellulose or mannitol, or a mixture thereof, wherein the weight ratio of microcrystalline cellulose to mannitol in the mixture is selected from 1.5:1 to 2.5:1;
   2.0-4.0% wt of hydroxypropyl cellulose or polyvinylpyrrolidone, or a mixture thereof;
   0.03-2.0% wt of sodium dodecyl sulfate or magnesium dodecyl sulfate, or a mixture thereof;
   2.0-4.0% wt of sodium carboxymethyl starch or croscarmellose sodium, or a mixture thereof; and optionally, 0.5-1% wt of magnesium stearate or colloidal silicon dioxide, or a mixture thereof; or
D) 3.0-5.0% wt of the compound of formula **I** or the compound of formula **II;**
   50-70% wt of microcrystalline cellulose;
   20-40% wt of mannitol;
   2.0-6.0% wt of hydroxypropyl cellulose;
   0.05-0.2% wt of sodium dodecyl sulfate;
   2.0-6.0% wt of croscarmellose sodium; and
   optionally, 0.5-2% wt of magnesium stearate; or
E) 3.4-4.6% wt of the compound of formula **I** or the compound of formula **II;**
   55-60% wt of microcrystalline cellulose;
   26-32% wt of mannitol;
   2.0-4.0% wt of hydroxypropyl cellulose;
   0.1-0.5% wt of sodium dodecyl sulfate;
   2.0-4.0% wt of croscarmellose sodium; and
   optionally, 0.5-1% wt of magnesium stearate; or
F) 4.2% wt of the compound of formula **I** or the compound of formula **II;**
   58.3% wt of microcrystalline cellulose;
   29.4% wt of mannitol;
   4.0% wt of hydroxypropyl cellulose;
   0.1% wt of sodium dodecyl sulfate;
   3.0% wt of croscarmellose sodium; and
   optionally, 1.0% wt of magnesium stearate.

In some embodiments of the present application, the compound of formula **I** or the compound of formula **II** is present in the form of a free base or a hydrochloride salt. In some typical embodiments of the present application, the compound of formula **I** or the compound of formula **II** is present in the form of a free base. In some embodiments of the present application, the compound of formula **I** or the compound of formula **II** is present in the form of a monohydrochloride salt.

In some embodiments of the present application, the solid pharmaceutical composition further comprises a coating agent. In some embodiments, the coating agent is formed from an aqueous film coating composition, wherein the aqueous film coating composition comprises a film-forming polymer, water and/or an alcohol as a carrier, and optionally one or more auxiliary agents such as additives known in the film coating field. In some embodiments, the coating agent is selected from the group consisting of hydroxypropyl methylcellulose, hydroxypropyl cellulose, methyl cellulose, ethyl cellulose, hydroxyethyl cellulose, cellulose acetate phthalate, sodium ethylcellulose sulfate, carboxymethyl cellulose, polyvinylpyrrolidone, zein, acrylic polymers (for example, methacrylic acid/methacrylate copolymers, such as methacrylic acid/methyl methacrylate copolymers) and polyvinyl alcohol. In some typical embodiments, the coating agent comprises polyvinyl alcohol.

In some embodiments of the present application, the dissolution rate of the solid pharmaceutical composition within 30 minutes is not less than 60% of the labeled amount. In some embodiments of the present application, the dissolution rate of the solid pharmaceutical composition within 30 minutes is not less than 80% of the labeled amount. In some embodiments of the present application, the dissolution rate of the solid pharmaceutical composition within 30 minutes in a hydrochloric acid solution (pH 1.0), an acetate buffer (pH 4.5), a phosphate buffer (pH 6.8) or purified water is not less than 80% of the labeled amount, preferably not less than 85% of the labeled amount. In some embodiments of the present application, the dissolution rate of the solid pharmaceutical composition within 30 minutes in purified water is not less than 80% of the labeled amount, preferably not less than 85% of the labeled amount, more preferably not less than 90% of the labeled amount. In some typical embodiments of the present application, the dissolution rate of the solid pharmaceutical composition within 15 minutes in purified water is not less than 85% of the labeled amount.

In some embodiments of the present application, the dosage form of the solid pharmaceutical composition can be selected from the group consisting of powders, granules, tablets, capsules, pills, pellets, dispersions and inhalable powders, preferably tablets and capsules, more preferably tablets. In some embodiments of the present application, the solid pharmaceutical composition is a pharmaceutical composition in unit dose, and the amount of the compound of formula **I** or the compound of formula **II** per unit dose is 5-20 mg. In some embodiments of the present application, the amount of the compound of formula **I** or the compound of formula **II** per unit dose is 5 mg, 8 mg, 10 mg, 12 mg, 15 mg, 18 mg or 20 mg, or any range defined by endpoints of any of the foregoing values, or any value in the range.

In another aspect, the present application provides a method for preparing a solid pharmaceutical composition of the compound of formula **I** or the compound of formula **II.** In the present application, wet granulation can be used for preparing solid granules. In the wet granulation process, if necessary, additional excipients compatible with the compound of formula **I** or the compound of formula **II** may be added. The wet granulation can be conducted in a wet granulator (mixing granulation) or on a fluidized bed (fluidized bed granulation), preferably on a fluidized bed. In wet granulation, the compound of formula **I** or the compound of formula **II** can be added to a premix as a solid additive together with other additives or prepared into a solution (suspension or clear solution) for granulation. Preferably, it is prepared into a suspension, which is incorporated into the granules at the granulation stage.

In some embodiments of the present application, the method for preparing the solid pharmaceutical composition of the compound of formula **I** or the compound of formula **II** comprises:
1) mixing a diluent with a disintegrant to give a mixture for further use; mixing the compound of formula **I** or the compound of formula **II,** a binder, and a wetting agent with water to give a mixed solution;
2) spraying the mixed solution from step 1) to the mixture from step 1), granulating and drying through a fluidized bed, and sizing the resultant mixture to give dried granules; and
3) optionally, mixing the dried granules with a lubricant, and tableting;
wherein the mixed solution in step 1) is a suspension or a clear solution, preferably a suspension; and the diluent, binder, wetting agent, disintegrant and lubricant are as described above.

In some embodiments of the present application, in step 2), the inlet air temperature is selected from 35-90 °C, preferably 45-85 °C, more preferably 55-80 °C. In some embodiments of the present application, in step 2), the spraying pressure is selected from 400-1200 mbar, preferably 500-1100 mbar, more preferably 600-1000 mbar. In some embodiments of the present application, in step 2), the material temperature is selected from 20-40 °C, preferably 25-35 °C. In some embodiments of the present application, in step 2), the mesh size of the sieve is Φ 0.4-1.5 mm, preferably Φ 0.5-1.4 mm, more preferably Φ 0.6-1.2 mm.

In another aspect, the present application provides use of the solid pharmaceutical composition of the compound of formula **I** or the compound of formula **II** in preparing a medicament for treating a disease mediated by JAK.

In another aspect, the present application provides a solid pharmaceutical composition of the compound of formula **I** or the compound of formula **II** for use in treating a disease mediated by JAK

In another aspect, the present application provides use of the solid pharmaceutical composition of the compound of formula **I** or the compound of formula **II** in treating a disease mediated by JAK

In another aspect, the present application provides a method for treating a disease mediated by JAK, comprising administering to a subject a therapeutically effective amount of the solid pharmaceutical composition of the compound of formula **I** or the compound of formula **II.**

In some embodiments of the present application, the disease mediated by JAK includes, but is not limited to, tumors. In some embodiments, the tumor described herein is lymphoma or leukemia. The lymphoma described herein includes but is not limited to: Hodgkin's disease and non-Hodgkin's lymphoma, and the non-Hodgkin's lymphoma includes, but is not limited to: B-cell lymphoma and T-cell lymphoma. The leukemia described herein includes, but is not limited to: acute lymphoblastic leukemia, chronic lymphocytic leukemia, acute myeloid leukemia, and chronic myelocytic leukemia.

In some embodiments of the present application, the disease mediated by JAK is myeloproliferative neoplasm.

In some embodiments of the present application, myeloproliferative neoplasm includes polycythemia vera, thrombocythemia, and myelofibrosis. Polycythemia vera includes hydroxyurea and/or interferon resistant and/or intolerant polycythemia vera. Thrombocythemia includes essential thrombocythemia, and hydroxyurea and/or interferon resistant and/or intolerant essential thrombocythemia. Myelofibrosis includes primary myelofibrosis, post-polycythemia vera myelofibrosis (PPV-MF), and post-essential thrombocythemia myelofibrosis (PET-MF).

In some embodiments of the present application, myeloproliferative neoplasm and the diseases included thereof include low-risk, medium-risk, and high-risk myeloproliferative neoplasm. For example, polycythemia vera may include low-risk and high-risk polycythemia vera; thrombocythemia may include very low-risk, low-risk, medium-risk and high-risk thrombocythemia; myelofibrosis may include low-risk, medium-risk and/or high-risk myelofibrosis. In some embodiments, the polycythemia vera is a medium-risk and/or high-risk polycythemia vera, the thrombocythemia is a medium-risk and/or high-risk thrombocythemia, the myelofibrosis is a medium-risk and/or high-risk myelofibrosis, and the essential thrombocythemia is a medium-risk and/or high-risk essential thrombocythemia. For example, primary myelofibrosis, post-polycythemia vera myelofibrosis, and post-essential thrombocythemia myelofibrosis all belong to the corresponding medium- or high-risk myelofibrosis. In the present application, for the diagnostic criteria and risk stratification (for example, very low-risk, low-risk, medium-risk and high-risk) of the myeloproliferative neoplasm, general principles in the field may be referred to. It can be evaluated with reference to the NCCN Guidelines Version 2.2019 Myeloproliferative Neoplasms, for example, Dynamic International Prognostic Scoring System (DIPSS). In some embodiments, WHO 2016 and IWG-MRT are used as the diagnostic criteria.

In the present application, the myeloproliferative neoplasm includes mutant myeloproliferative neoplasms, and the mutant genes include, but are not limited to: JAK2 (e.g., JAK2 V617F), MPL, CALR, ASXL1/SRSF2/IDH1/21, JAK2 exon 12, TP53, SH2B3/IDH2/U2AF1/SF3B1/EZH2/TP53, MPL W515L/K, CALR Type 1/Type 1-like, triple-negative (no JAK2, MPL and CALR mutations), ASXL1, EZH2, IDH1/2, SRSF2, SF3B1, CALR/ASXL1, TP53, and U2AF1 Q157.

In some embodiments of the present application, the compound of formula **I** or the compound of formula **II** is administered in the form of a free base. In some embodiments of the present application, the compound of formula **I** or the compound of formula **II** is administered in the form of a crystalline free base. In some embodiments of the present application, the crystalline free base of the compound of formula **II** can be selected from crystalline form A and crystalline form B disclosed in WO2017215630.

In some embodiments of the present application, the compound of formula **II** is administered in the form of a hydrochloride salt. In some embodiments of the present application, the hydrochloride salt of the compound of formula **II** can be selected from the hydrochloride salts disclosed in WO2017101777.

The compound, or the stereoisomer or the pharmaceutically acceptable salt thereof disclosed herein can be administered by various routes, including but not limited to: oral, parenteral, intraperitoneal, intravenous, intra-arterial, transdermal, sublingual, intramuscular, rectal, transbuccal, intranasal, inhalational, vaginal, intraocular, topical, subcutaneous, intralipid, intra-articular and intrathecal administrations. In one specific embodiment, it is administered orally.

The amount of the compound, the stereoisomer or the pharmaceutically acceptable salt thereof, or the solid pharmaceutical composition thereof disclosed herein can be determined on the basis of severity of the disease, response, any treatment-related toxicity, and age and health status of the subject. For example, it can be determined on the basis of the subject's routine blood test, which includes platelet count, neutrophil count, and hemoglobin concentration, etc. In some embodiments, the daily dose for administering the compound, the stereoisomer or the pharmaceutically acceptable salt thereof, or the solid pharmaceutical composition thereof disclosed herein is 1 mg to 100 mg. In some embodiments, the daily dose for administering the compound, the stereoisomer or the pharmaceutically acceptable salt thereof, or the solid pharmaceutical composition thereof disclosed herein can be selected from the group consisting of 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg and 100 mg, or any range defined by endpoints of any of the foregoing values, or any value in the range, for example, 1 mg to 90 mg, 5 mg to 80 mg, 10 mg to 70 mg, 15 mg to 60 mg and 20 mg to 50 mg, etc. In some specific embodiments, the daily dose for administering the compound, the stereoisomer or the pharmaceutically acceptable salt thereof, or the solid pharmaceutical composition thereof disclosed herein can be selected from 1 mg to 50 mg, 5 mg to 50 mg, 5 mg to 45 mg, 5 mg to 40 mg, 10 mg to 35 mg, or 10 mg to 30 mg. In some specific embodiments, the daily dose for administering the compound, the stereoisomer or the pharmaceutically acceptable salt thereof, or the solid pharmaceutical composition thereof disclosed herein can be selected from the group consisting of 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg and 50 mg, or any range defined by endpoints of any of the foregoing values, or any value in the range, for example, 2 mg to 50 mg, 10 mg to 40 mg, 5 mg to 30 mg and 5 mg to 20 mg, etc.

The compound, the stereoisomer or the pharmaceutically acceptable salt thereof, or the solid pharmaceutical composition thereof disclosed herein can be administered once or multiple times a day. In some embodiments, the compound, the stereoisomer or the pharmaceutically acceptable salt thereof, or the solid pharmaceutical composition thereof disclosed herein can be administered once or twice a day. The compound, or the stereoisomer or the pharmaceutically acceptable salt thereof can also be administered in a unit dosage form. In one embodiment, it is administered in a unit dosage form once or twice daily. In one embodiment, it is administered in a unit dosage form of an oral solid formulation once or twice daily.

The route of administration can be determined according to factors such as the activity and toxicity of the drug, and tolerance of the subject. In some embodiments, the compound, or the stereoisomer or the pharmaceutically acceptable salt thereof is administered in an intermittent regimen.

The compound, the stereoisomer or the pharmaceutically acceptable salt thereof, or the solid pharmaceutical composition thereof disclosed herein can be administered in an intermittent regimen. The intermittent regimen includes treatment periods and interruption periods. During the treatment periods, the compound, the stereoisomer or the pharmaceutically acceptable salt thereof, or the solid pharmaceutical composition thereof may be administered once, twice or multiple times a day. The intermittent regimen can be given on the basis of severity of the disease, response, any treatment-related toxicity, and age and health status of the subject. For example, it can be given on the basis of the patient/subject's routine blood test, which includes platelet count, neutrophil count, and hemoglobin concentration, etc.

### Technical Effects

The compound disclosed herein can effectively shrink the spleen of a subject. The compound disclosed herein has a good therapeutic effect on myeloproliferative neoplasm and has superior safety.

The solid pharmaceutical composition disclosed herein has excellent stability and dissolution properties, and is suitable for clinical use. Further, the tablet solid pharmaceutical composition of the present application has the property of rapid release, with a dissolution rate within 30 minutes not less than 80% of the labeled amount.

### Definitions and Description

Unless otherwise stated, the following terms used in the present application shall have the following meanings. A specific term, unless otherwise specifically defined, should not be considered uncertain or unclear, but construed according to its ordinary meaning in the field. When referring to a trade name, it is intended to refer to its corresponding commercial product or its active ingredient. The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed by basic radicals and free acids and salts formed by acidic radicals and free bases, for example, hydrochloride, hydrobromide, nitrate, sulfate, phosphate, formate, acetate, trifluoroacetate, fumarate, oxalate, maleate, citrate, succinate, mesylate, benzenesulfonate and *p*-methylbenzenesulfonate, preferably, hydrochloride, hydrobromide, sulfate, formate, acetate, trifluoroacetate, fumarate, maleate, mesylate, *p*-methylbenzenesulfonate, sodium salt, potassium salt, ammonium salt, and amino acid salt, etc. In the present application, when forming a pharmaceutically acceptable salt, the free acid and the basic radical are in a molar ratio of about 1:0.5 to 1:5, preferably, 1:0.5, 1:1, 1:2, 1:3, 1:4, 1:5, 1:6, 1:7 or 1:8. In the present application, when forming a pharmaceutically acceptable salt, the free base and the acidic radical are in a molar ratio of about 1:0.5 to 1:5, preferably, 1:0.5, 1:1, 1:2, 1:3, 1:4, 1:5, 1:6, 1:7 or 1:8.

As used herein, if the compound of formula **I** or the compound of formula **II** has, for example, at least one basic site, an acid addition salt can be formed. If needed, it can further form an acid addition salt with additional basic sites. A compound with at least one acidic group (for example, -COOH) can further form a salt with a base. A compound, for example, comprising both carboxyl and amino, can further form an inner salt.

The compound disclosed herein can be asymmetrical, for example, has one or more stereoisomers. Unless otherwise stated, all stereoisomers are included, for example, enantiomers and diastereoisomers. The compound containing asymetrical carbon atoms disclosed herein can be separated in an optically pure form or in a racemic form. The optically pure form can be separated from a racemic mixture or can be synthesized using a chiral raw material or a chiral reagent.

The term "subject" refers to a mammal. In some embodiments, the subject is a human.

The term "pharmaceutical composition" refers to a mixture consisting of one or more of the compounds or pharmaceutically acceptable salts thereof disclosed herein and a pharmaceutically acceptable excipient. The pharmaceutical composition is intended to facilitate the administration of the compound to a subject.

The terms "treat", "treating", and "treatment" refer to administering the compound or pharmaceutical composition disclosed herein to ameliorate or eliminate a disease or one or more symptoms associated with the disease, and includes:
(i) inhibiting a disease or disease state, i.e., arresting its development; and
(ii) alleviating a disease or disease state, i.e., causing its regression.

The term "effective amount" refers to an amount of the compound disclosed herein for (i) treating a specific disease, condition or disorder; (ii) alleviating, relieving or eliminating one or more symptoms of a specific disease, condition or disorder, or (iii) preventing or delaying onset of one or more symptoms of a specific disease, condition or disorder described herein. The amount of the compound disclosed herein composing the "therapeutically effective amount" varies dependently on the compound, the disease state and its severity, the administration regimen, and the age of the mammal to be treated, but can be determined routinely by those skilled in the art in accordance with their knowledge and the present disclosure.

In the present application, the "pharmaceutically acceptable salt" includes, but is not limited to: acid addition salts of inorganic acids such as hydrochloric acid, hydrobromic acid, sulfuric acid, nitric acid or phosphoric acid; or acid addition salts of organic acids such as formic acid, acetic acid, trifluoroacetic acid, succinic acid, malic acid, maleic acid, fumaric acid, oxalic acid, tartaric acid, citric acid, methanesulfonic acid, benzenesulfonic acid, or *p*-toluenesulfonic acid; or acid addition salts of acidic amino acids such as aspartic acid or glutamic acid. The solvates include, but are not limited to, hydrates.

In the present application, the amount of the compound of formula **I** or the compound of formula **II** in the pharmaceutical composition is determined on a basis of free base form.

The diluent described herein is also referred to as filler, and is mainly classified into water-soluble diluents, water-insoluble diluents, diluents for direct compression, etc., including, but not limited to: starch, sucrose, dextrin, lactose, pregelatinized starch, microcrystalline cellulose, inorganic salts and/or sugar alcohols. The lactose includes, but is not limited to: anhydrous lactose and lactose monohydrate, or a mixture thereof.

According to sources, the binder described herein may be classified into natural binders and synthetic binders. According to use, the binder can also be classified into the binders only demonstrating viscosity in aqueous solutions or mucilage, binders demonstrating viscosity in the dry state, and binders demonstrating viscosity after being dissolved or wetted by a non-aqueous solvent. The binders include, but are not limited to: starch slurry, cellulose derivatives, polyvidone, gelatin, polyethylene glycol, sucrose solutions and/or sodium alginate solutions.

The wetting agent described herein includes liquids and/or surfactants with low surface tension and water miscibility. The surfactants include anionic surfactants, cationic surfactants, zwitterionic surfactants and/or nonionic surfactants. The anionic surfactants include, but are not limited to: alkylbenzene sulfonate salts, alkylsulfonate ester salts, alkyl sulfonate salts, alkyl sulfate salts, fluorinated fatty acid salts, polysiloxanes and/or fatty alcohol sulfate salts. The cationic surfactants include, but are not limited to: quaternary ammonium compounds, alkylpyridinium salts and/or amine salts. The zwitterionic surfactants include, but are not limited to: lecithin, amino acids and/or betaines; the nonionic surfactants include, but are not limited to: alkyl polyglycosides (APGs), fatty acid glycerides, sorbitan fatty acid esters (Span), polysorbates (Tween), polyoxyethylenes and/or poloxamers.

The disintegrant described herein includes, but is not limited to: starch and derivatives thereof, celluloses, surfactants, effervescent disintegrants, gums, alginates and/or ion exchange resins.

The lubricant described herein broadly includes three excipients: lubricants (in a narrow sense), glidants and antiadherents. The lubricant described herein includes, but is not limited to: stearates, colloidal silicon dioxide, talc, hydrogenated vegetable oil and/or polyethylene glycols.

The amount "% wt" of a certain component (including active substances or excipients) used herein refers to the percentage of the weight of the component based on the total weight of the solid pharmaceutical composition (the weight of the compound of formula **I** and the compound of formula **II** is calculated in the free base form). The total weight of the solid pharmaceutical composition does not include the weight of the coating agents.

The preparation of the solid pharmaceutical compositions or the corresponding dosage forms disclosed herein can be conducted according to methods well known in the art. Specifically, the preparation method may comprises procedures of pulverizing, mixing, sieving, granulating, filling, tableting, etc. The required steps and the method or device for implementing the specific procedures are selected according to the practicalities. For example, the pulverizing procedure may be performed by a mortar, a ball mill, a roller press, an impact mill, a hammer mill and/or a jet mill; the mixing procedure may be agitation mixing, grinding mixing and/or sieving mixing; the sieving procedure may be performed by a sieve shaker and/or a vibrating screen; or referring to Pharmacy (6th or 7th Edition, People's Medical Publishing House) edited by Cui Fude et al.

The term "or a mixture thereof" means a mixture of two or more, for example, "the diluent is selected from the group consisting of microcrystalline cellulose, mannitol, lactose, sucrose, starch, pregelatinized starch and dextrin, or a mixture thereof" means that the diluent is selected from the group consisting of microcrystalline cellulose, mannitol, lactose, sucrose, starch, pregelatinized starch and dextrin, or a mixture of two or more.

The term "labeled amount" in the field of pharmaceutical compositions refers to the content of the specified active substance in a unit dose of the formulation.

The compound of formula **I** and the compound of formula **II** disclosed herein can be prepared with reference to the preparation methods in WO2016095805 or WO2017215627.

### DETAILED DESCRIPTION

The present invention will be illustrated in more detail through specific examples. The following examples are provided for illustrative purposes only, and are not intended to limit the present invention in any way.

### Example 1 (3R)-3-{3-amino-4-{7H-pyrrolo[2,3-d]pyrimidin-4-yl}-1H-pyrazol-1-yl}-3-cyclopentylpropionitrile (II)

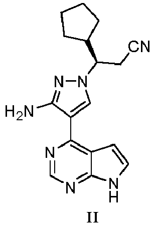

The title compound was prepared with reference to WO2016095805 or WO2017215627.

### Example 2 Solid pharmaceutical composition tablets of (3R)-3-{3-amino-4-{7H-pyrrolo[2,3-d] pyrimidin-4-yl}-1H-pyrazol-1-yl}-3-cyclopentylpropionitrile (II)

The formulations of 5 mg and 20 mg solid pharmaceutical composition tablets were shown in Table 1:

**Table 1. Formulations of 5 mg and 20 mg tablets**

| **Component** | **Formulation (mg)** | | **Percentage (% wt)** |
|---|---|---|---|
| Compound of formula **II** | 5 | 20 | 4.2 |
| Mannitol | 35.275 | 141.1 | 29.4 |
| Microcrystalline cellulose | 70 | 280 | 58.3 |
| Croscarmellose sodium | 3.6 | 14.4 | 3 |
| Sodium dodecyl sulfate | 0.125 | 0.5 | 0.1 |
| Hydroxypropyl cellulose | 4.8 | 19.2 | 4 |
| Magnesium stearate | 1.2 | 4.8 | 1 |
| Purified water | Proper amount | Proper amount | - |

Procedures:
1) Mannitol, microcrystalline cellulose and croscarmellose sodium were mixed to prepare a mixture A for further use;
   Preparation of drug substance suspension: Hydroxypropyl cellulose was dissolved in purified water to prepare a 4% (w/w) hydroxypropyl cellulose solution; sodium dodecyl sulfate was dissolved; the compound of formula **II** was added and dispersed by stirring to prepare a drug substance suspension;
2) Fluidized bed granulation and drying: the drug substance suspension was applied to the mixture A by spraying for fluidized granulation. Granulation parameters: inlet air temperature: 55-80 °C, spraying pressure: 600-1000 mbar, material temperature: 25-35 °C. Drying started after the spraying, and ended when the material temperature was higher than 45 °C. The material was sized in a mill through a sieve with a mesh size of Φ 0.6-1.2 mm, and dried granules were obtained;
3) The dried granules and magnesium stearate were sequentially fed into a hopper mixer and well mixed to give a solid pharmaceutical composition for tableting.

### Example 3 Preparation of solid pharmaceutical compositions of formulations A-I

The solid pharmaceutical compositions of formulations A-I were prepared in 5 mg tablets with reference to the method of Example 2. The specific formulations were as follows:

**Table 2. Solid pharmaceutical compositions of formulations A-E**

| **Materials** | **Percentage (% wt)** | | | | |
|---|---|---|---|---|---|
| | Formulation | Formulation | Formulation | Formulation | Formulation |
| | A | B | C | D | E |
| Compound of formula **II** | 4.2 | 4.2 | 4.2 | 4.2 | 4.2 |
| Mannitol | 31.0 | 30.9 | 30.5 | 30.9 | 30.9 |
| Microcrystalline cellulose | 58.3 | 58.3 | 58.3 | 58.3 | 58.3 |
| Croscarmellose sodium | 3 | 3 | 3 | 2 | 4 |
| Hydroxypropyl cellulose | 3 | 3 | 3 | 4 | 2 |
| Sodium dodecyl sulfate | 0.0 | 0.1 | 0.5 | 0.1 | 0.1 |
| Magnesium stearate | 0.5 | 0.5 | 0.5 | 0.5 | 0.5 |
| Total | 100 | 100 | 100 | 100 | 100 |

**Table 3. Solid pharmaceutical compositions of formulations F-I**

| **Materials** | **Percentage (% wt)** | | | |
|---|---|---|---|---|
| | Formulation F | Formulation G | Formulation H | Formulation I |
| Compound of formula **II** | 4.2 | 4.2 | 4.2 | 4.2 |
| Mannitol | 44.65 | 44.15 | 28.7 | 30.4 |
| Microcrystalline cellulose | 44.65 | 44.15 | 57.6 | 60.9 |
| Croscarmellose sodium | 2 | 5 | 5 | 2 |
| Hydroxypropyl cellulose | 4 | 2 | 4 | 2 |
| Sodium dodecyl sulfate | 0 | 0 | 0 | 0 |
| Magnesium stearate | 0.5 | 0.5 | 0.5 | 0.5 |
| Total | 100 | 100 | 100 | 100 |

### Example 4 Stability test of solid pharmaceutical compositions

According to the *Guidelines for the Stability Test of APIs and Preparations* of China, the stability of the solid pharmaceutical compositions of formulations disclosed above was investigated. The 5 mg and 20 mg solid pharmaceutical composition tablets obtained in Example 2 were placed in open conditions in illumination (6000 lux), and at high temperature (60 °C) and high humidity (RH75%). A proper amount of the sample was added into water-acetonitrile (30:70) to prepare a test solution containing about 0.5 mg of the test sample per 1 mL; octadecylsilane chemically bonded to silica gel was used as the filler; linear gradient elution was performed using potassium dihydrogen phosphate buffer-acetonitrile (90:10) and acetonitrile as mobile phase A and mobile phase B, respectively; the flow rate was 1.0 mL per minute; the detection wavelength was 220 nm; the column temperature was 30 °C. The total impurity content was assayed and the results were as follows:

**Table 4. Stability test**

| Formulation | Total impurities (%) | | | |
|---|---|---|---|---|
| | Day 0 | 60 °C for 30 days | 75% humidity for 30 days | Illumination (6000 lux, 0.9 w/m²) for 10 days |
| 5 mg | 0.15 | 0.26 | 0.16 | 0.23 |
| 20 mg | 0.11 | 0.27 | 0.15 | 0.15 |

### Example 5 Dissolution property test

According to the second method for dissolution and release tests of Chapter 0931 in *Chinese Pharmacopoeia* (Volume IV, 2015 Edition), the dissolution performance of the solid pharmaceutical compositions of the above formulations in four media was investigated. The four media were hydrochloric acid solution (pH 1.0), acetate buffer (pH 4.5), phosphate buffer (pH 6.8) and purified water, respectively, with a volume of 900 mL. A reference solution containing about 5.6 µg of the compound of formula **II** per 1 mL was prepared. The method and conditions were: paddle method, rotation speed: 50 rpm, medium temperature: 37 °C ± 0.5 °C, sampling time points: 5 min, 10 min, 15 min, 20 min, 30 min and 45 min. Octadecylsilane chemically bonded to silica gel was used as a filler; potassium dihydrogen phosphate buffer-acetonitrile (70:30) was used as the mobile phase; the flow rate was 0.4 mL per minute; the detection wavelength was 220 nm; the column temperature was 30 °C. The results were as follows:

**Table 5. Dissolution property test of 5 mg tablet of Example 2**

| **Medium** | **Cumulative dissolution (%)** | | | | |
|---|---|---|---|---|---|
| | 5min | 10min | 15min | 30min | 45min |
| Hydrochloric acid, pH 1.0 | 97 | 100 | 102 | 102 | 102 |
| Acetate buffer, pH 4.5 | 82 | 95 | 98 | 100 | 101 |
| Phosphate buffer, pH 6.8 | 49 | 74 | 85 | 95 | 98 |
| Purified water | 83 | 96 | 101 | 101 | 101 |

**Table 6. Dissolution property test of 20 mg tablet of Example 2**

| **Medium** | **Cumulative dissolution (%)** | | | | |
|---|---|---|---|---|---|
| | 5min | lOmin | 15min | 30min | 45min |
| Hydrochloric acid, pH 1.0 | 91 | 95 | 96 | 98 | 99 |
| Acetate buffer, pH 4.5 | 60 | 79 | 86 | 93 | 96 |
| Phosphate buffer, pH 6.8 | 49 | 68 | 76 | 86 | 91 |
| Purified water | 74 | 85 | 89 | 95 | 96 |

**Table 7 Dissolution property test of formulations A-E**

| **Medium: purified water Time** | **Cumulative dissolution (%)** | | | | |
|---|---|---|---|---|---|
| | Formulation A | Formulation B | Formulation C | Formulation D | Formulation E |
| 5min | 44 | 71 | 59 | 67 | 73 |
| 10min | 54 | 83 | 80 | 81 | 79 |
| 15min | 60 | 90 | 92 | 92 | 88 |
| 30min | 63 | 92 | 93 | 94 | 90 |

**Table 8 Dissolution property test of formulations F-I**

| **Medium: phosphate buffer, pH 6.8 Time** | **Cumulative dissolution (%)** | | | |
|---|---|---|---|---|
| | Formulation F | Formulation G | Formulation H | Formulation I |
| 5min | 39 | 44 | 44 | 45 |
| 10min | 51 | 58 | 58 | 58 |
| 15min | 56 | 63 | 62 | 63 |
| 30min | 63 | 69 | 68 | 68 |

### Example 6 Dosage and compound

### 6.1 Regimen

The medicament used was the 5 mg tablet of Example 2.

Administration: oral administration at fasting, once a day during pre-tests. Sequentially, for treatment groups the medicament was administered orally every 12 hours, and the subjects were deprived of food within 2 hours after administration. The treatment was given in 28-day cycle. Subjects enrolled should be treated for at least 1 cycle and subjected to tolerability observation and preliminary efficacy observation. For subjects with investigator-assessed clinical beneficiaries and agreeing to continue the investigational treatment, the treatment should continue for free until the disease progressed or the investigator determined that it was not suitable to continue the investigational treatment. After the enrollment was completed, the study was closed when subjects received the treatment for 6 consecutive cycles.

### 6.2 Enrollment criteria

1) Aged ≥ 18 years; ECOG scoring: 0-2; an expected survival time of more than 3 months;
2) Diagnosed with PMF, PV, ET, PPV-MF or PET-MF according to the PMF, PV and ET criteria issued by the World Health Organization (WHO) in 2016 and the PPV-MF and PET-MF criteria recommended by the International Working Group-Myeloproliferative Neoplasms Research and Treatment (IWG-MRT);
3) Subjects with medium-risk-1 myelofibrosis or above under treatment according to the Dynamic International Prognostic Scoring System (DIPSS).
4) PV or ET subjects resistant or intolerant to hydroxyurea and/or interferons;
5) The main hematology laboratory test results meet the following criteria:
   Platelet count (PLT) > 100 × 10⁹/L;
   Absolute neutrophil count (ANC) > 1.5 × 10⁹/L;
   Hemoglobin concentration (Hgb) > 75 g/L (no blood products such as whole blood, red blood cell suspensions or the like have been infused within four weeks);
6) The most prominent part of the subject's spleen being ≥ 5 cm away from the lower edge of the rib;
7) Bone marrow blast cells and peripheral blood blast cells < 20%;
8) The main liver and kidney laboratory test results meet the following criteria:
   Alanine transaminase (ALT) and aspartate transaminase (AST) ≤ 2.5 × upper limit of normal (ULN);
   Serum creatinine ≤ 2.0 mg/dL (176.82 µmol/L);
   Direct bilirubin ≤ 2 × ULN;
9) The main coagulation laboratory test results meet the following criteria:
   Prothrombin time (PT) or thrombin time (TT) is within a range of normal range ± 3 s;
   Activated partial thromboplastin time (APTT) is within a range of normal range ± 10 s;
10) 2 weeks or more from other MPN treatment; 4 weeks or more from MPN surgery;
11) Female subjects should agree to take contraceptive measures (such as intrauterine devices, contraceptives or condoms) during the study and for at least 6 months after the study; serum pregnancy test results should be negative within 7 days before the study, and the subjects must not be breastfeeding; male subjects should agree to take contraceptive measures during the study and for at least 6 months after the study;
12) Voluntary participation.

### 6.3 Exclusion criteria

Subjects who meet any of the following items will not be enrolled in the study:
1) Having other malignant tumors within 5 years except for cured non-melanocytic tumor, skin cancer and carcinoma *in situ;*
2) Participation in clinical trials of other drugs within four weeks;
3) Factors affecting oral administration (such as inability to swallow, gastrointestinal resection, chronic diarrhea and intestinal obstruction, etc.);
4) A history of psychotropic drug abuse and difficulties in rehabilitation, or having mental disorders;
5) HBsAg positive; HBcAb positive and having a HBV-DNA test result ≥ ULN; HCV antibody positive; HIV antibody positive;
6) Immunodeficiency (acquired or congenital immunodeficiency diseases, or having a history of organ transplantation);
7) Having arterial/venous thrombosis events within 4 weeks, such as cerebrovascular accidents (including transient ischemic attacks), deep vein thrombosis and pulmonary embolism;
8) Receiving long-term (≥3 days) high-dose (≥10 mg equivalent dose of prednisone) glucocorticoid or other immunosuppressive therapy within 28 days before the screening visit, or dose increase;
9) Systemic active infections such as bacterial, fungal, parasitic or viral infections that need to be treated and have clinical significance. For those who need antibiotics to treat acute bacterial infections, the screening/enrollment should be postponed until the end of the antibiotic treatment;
10) Significant cardiovascular diseases such as heart failure graded 2 and above by the New York Heart Association (NYHA), unstable angina pectoris, myocardial ischemia or myocardial infarction in the past 3 months, arrhythmia (for females QTc > 470 ms, for males QTc > 450 ms) and grade I cardiac insufficiency;
11) Hypertension (systolic blood pressure ≥ 150 mmHg, diastolic blood pressure ≥ 100 mmHg) that cannot be controlled by medication;
12) Other concomitant disease that seriously affects the safety of the subject or the completion of the study as determined by the investigator.

### 6.4 Blood sampling schedule

Blood samples were collected by an indwelling catheter in the vein of the upper extremity of the subjects, and about 3 mL of venous blood was collected at each time points. The blood samples were collected in pre-treated blood collection tubes with heparin sodium for anticoagulation (no more than 0.5 h at room temperature), centrifuged at 4 °C for 10 min (2500g), separated to give the plasma, and stored at -80 °C for testing (at room temperature for no more than 2 h at room temperature).

### 6.5 Observations

From the second cycle of continuous treatment:
1) A comprehensive physical examination was performed at each visit: weight, vital signs, physical examination of various organs;
2) Various clinical manifestations, symptoms and signs (including hepatomegaly and splenomegaly) during the treatment period were observed, and symptom burden of the subjects was measured according to Myeloproliferative Tumor Total Symptom Assessment Scale every 3 cycles;
3) At the end of the second cycle, every 3 cycles from the third cycle, and withdrawal, the spleen MRI was performed.
4) At the end of the second cycle, every 3 cycles from the third cycle, and withdrawal, bone marrow puncture smear and peripheral blood smear differential counting was performed;
5) At the end of the second cycle, every 3 cycles from the third cycle, and withdrawal, bone marrow biopsy was performed for pathological cytological analysis and reticular fiber (silverophilic) staining.

### 6.6 Results and evaluation

**Table 9 Results and evaluation of subjects**

| Subject number | 002 | 003 | 004 | 006 | 007 | 008 | 009 | 010 |
|---|---|---|---|---|---|---|---|---|
| Administration route | 5mg bid | 5mg bid | 5mg bid | 10mg bid | 10mg bid | 10mg bid | 15mg bid | 15mg bid |
| Completed treatment cycle | 9 | 9 | 7 | 6 | 6 | 4 | 3 | 3 |
| Best change of spleen palpation (%) | -45% | -20% | -17% | -24% | -50% | -19% | -40% | -53% |
| Best change of spleen NMR volume (%) | -29% | -14% | -31% | -27% | Null | Null | Null | Null |

| | | | | | | | | |
|---|---|---|---|---|---|---|---|---|
| Note: Null means not available. | | | | | | | | |

## Claims

1. A compound of formula **I,** a stereoisomer or a pharmaceutically acceptable salt, or a pharmaceutical composition thereof for use in treating myeloproliferative neoplasm: 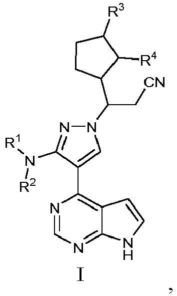
wherein, R¹ and R² are each independently selected from the group consisting of H, C₁₋₆ alkyl, C₁₋₆ alkylacyl and C₁₋₆ alkylsulfonyl;
R³ and R⁴ are each independently selected from the group consisting of H, hydroxyl and oxo; the pharmaceutical composition comprises the compound of formula **I,** or the stereoisomer or the pharmaceutically acceptable salt thereof.

2. The compound of formula **I** according to claim 1, wherein R¹ and R² are each independently selected from the group consisting of H, methyl, ethyl, propyl, butyl, pentyl, hexyl, formyl, acetyl, propanoyl, butyryl, valeryl, hexanoyl, methylsulfonyl, ethylsulfonyl, propylsulfonyl, butylsulfonyl, pentylsulfonyl and hexylsulfonyl; preferably, R¹ is H, and R² is selected from the group consisting of methyl, ethyl, propyl, butyl, pentyl, hexyl, formyl, acetyl, propanoyl, butyryl, valeryl, hexanoyl, methylsulfonyl, ethylsulfonyl, propylsulfonyl, butylsulfonyl, pentylsulfonyl and hexylsulfonyl.

3. The compound of formula **I** according to claim 1, wherein the compound of formula **I** has a configuration shown in the following formulas:

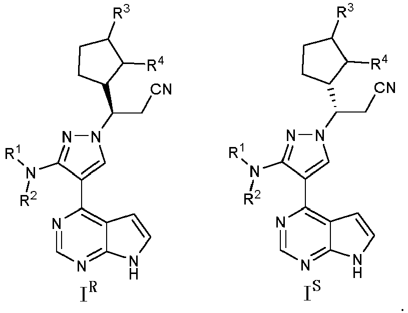

4. The compound of formula **I** according to claim 1, wherein the compound of formula **I** is selected from the group consisting of:

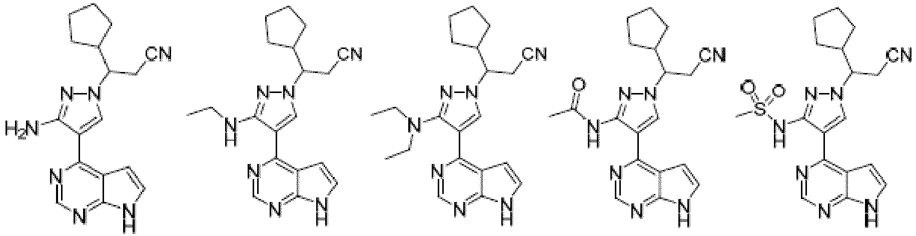

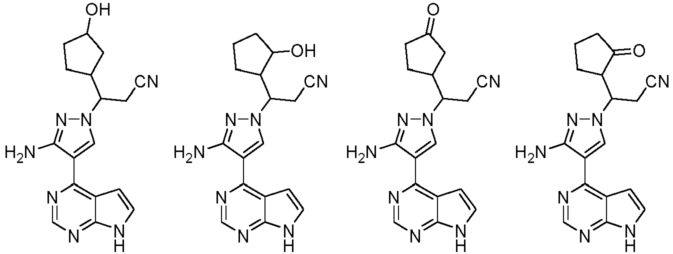

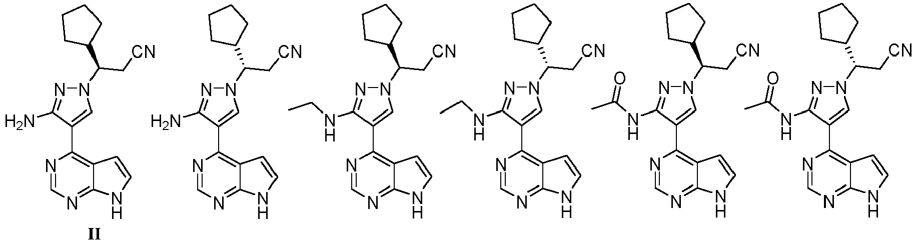

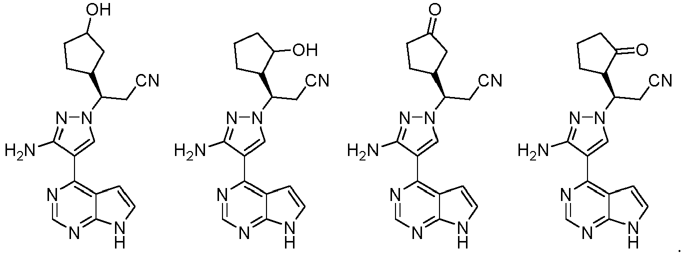

5. A method for treating myeloproliferative neoplasm, comprising administering to a subject an effective amount of a compound of formula **I,** a stereoisomer or a pharmaceutically acceptable salt, or a pharmaceutical composition thereof: 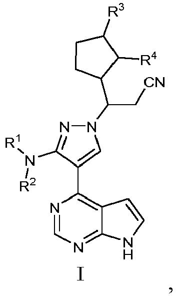
wherein, R¹ and R² are each independently selected from the group consisting of H, C₁₋₆ alkyl, C₁₋₆ alkylacyl and C₁₋₆ alkylsulfonyl;
R³ and R⁴ are each independently selected from the group consisting of H, hydroxyl and oxo; the pharmaceutical composition comprises the compound of formula **I,** or the stereoisomer or the pharmaceutically acceptable salt thereof.

6. A solid pharmaceutical composition comprising a compound of formula **I,** or a stereoisomer or a pharmaceutically acceptable salt thereof, and a diluent, a binder, a wetting agent and a disintegrant, 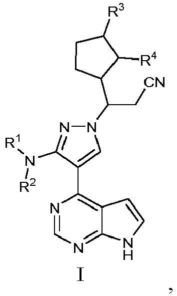
wherein, R¹ and R² are each independently selected from the group consisting of H, C₁₋₆ alkyl, C₁₋₆ alkylacyl and C₁₋₆ alkylsulfonyl;
R³ and R⁴ are each independently selected from the group consisting of H, hydroxyl and oxo.

7. The solid pharmaceutical composition according to claim 6, wherein R¹ and R² are each independently selected from the group consisting of H, methyl, ethyl, propyl, butyl, pentyl, hexyl, formyl, acetyl, propanoyl, butyryl, valeryl, hexanoyl, methylsulfonyl, ethylsulfonyl, propylsulfonyl, butylsulfonyl, pentylsulfonyl and hexylsulfonyl; preferably, R¹ is H, and R² is selected from the group consisting of methyl, ethyl, propyl, butyl, pentyl, hexyl, formyl, acetyl, propanoyl, butyryl, valeryl, hexanoyl, methylsulfonyl, ethylsulfonyl, propylsulfonyl, butylsulfonyl, pentylsulfonyl and hexylsulfonyl.

8. The solid pharmaceutical composition according to claim 6 or 7, wherein the amount of the compound of formula **I** is selected from 1-30% wt, preferably 1-25% wt, 1-20% wt, 2-20% wt, 2-15% wt, 2-10% wt, 3-10% wt, or 2-8% wt, more preferably 3-8% wt, further more preferably 3.5-6% wt.

9. The solid pharmaceutical composition according to any one of claims 6-8, wherein the diluent is selected from the group consisting of microcrystalline cellulose, mannitol, lactose, sucrose, starch, pregelatinized starch and dextrin, or a mixture thereof; preferably, microcrystalline cellulose, mannitol, lactose and pregelatinized starch, or a mixture thereof; more preferably, microcrystalline cellulose and mannitol, or a mixture thereof; and/or
the amount of the diluent is selected from 50-95% wt, preferably 60-95% wt, 65-95% wt, 70-95% wt, 75-95% wt, 80-95% wt, 80-90% wt or 85-95% wt, more preferably 85-90% wt.

10. The solid pharmaceutical composition according to any one of claims 6-9, wherein the diluent is a mixture of microcrystalline cellulose and mannitol, wherein the weight ratio of microcrystalline cellulose to mannitol is selected from 1:1 to 5:1, preferably 1:1 to 4:1, 1.2:1 to 3.5:1 or 1.2:1 to 3:1, more preferably 1.5:1 to 2.5:1.

11. The solid pharmaceutical composition according to any one of claims 6-10, wherein the binder is selected from the group consisting of hydroxypropyl methylcellulose, carboxymethyl cellulose, sodium carboxymethyl cellulose, ethyl cellulose, methyl cellulose, hydroxypropyl cellulose, low-substituted hydroxypropyl cellulose, gelatin, polyvinylpyrrolidone, partially hydrolyzed starch, starch, pregelatinized starch, sucrose, glucose, gelatin, polyethylene glycol and polyvinyl alcohol, or a mixture thereof; preferably hydroxypropyl methylcellulose, carboxymethyl cellulose, sodium carboxymethyl cellulose, ethyl cellulose, methyl cellulose, hydroxypropyl cellulose, low-substituted hydroxypropyl cellulose and polyvinylpyrrolidone, or a mixture thereof; more preferably hydroxypropyl cellulose and polyvinylpyrrolidone, or a mixture thereof; and/or
the amount of the binder is selected from 1.0-10% wt, preferably 1.0-8.0% wt, 1.0-6.0% wt or 1.0-5.0% wt, more preferably 2.0-4 0% wt.

12. The solid pharmaceutical composition according to any one of claims 6-11, wherein the wetting agent is selected from the group consisting of sodium dodecylbenzene sulfonate, magnesium dodecylbenzene sulfonate, sodium tetradecylbenzene sulfonate, sodium hexadecylbenzene sulfonate, sodium octadecylbenzene sulfonate, sodium dodecyl sulfonate, magnesium dodecyl sulfonate, sodium tetradecyl sulfonate, sodium hexadecyl sulfonate, sodium octadecyl sulfonate, sodium dodecyl sulfate, magnesium dodecyl sulfate, sodium tetradecyl sulfate, sodium hexadecyl sulfate, sodium octadecyl sulfate, sodium lauroyl sarcosine, sodium lactate, sodium palmitate, lauric isopropanolamide, lauric diethanolamide, tetradecyl lactate, hexadecyl lactate, polysorbate 20, polysorbate 40, polysorbate 60, polysorbate 65, polysorbate 80, polysorbate 85, polyoxyethylene lauryl ether, polyoxyethylene cetyl ether, polyoxyethylene sorbitol tetraoleyl ether, polyoxyethylene stearate, polyoxyethylene castor oil and polyoxyethylene hydrogenated castor oil, or a mixture thereof; preferably sodium dodecyl sulfonate, magnesium dodecyl sulfonate, sodium tetradecyl sulfonate, sodium dodecyl sulfate, magnesium dodecyl sulfate, sodium tetradecyl sulfate, sodium hexadecyl sulfate, sodium octadecyl sulfate and sodium lauroyl sarcosine, or a mixture thereof; more preferably sodium dodecyl sulfate and magnesium dodecyl sulfate, or a mixture thereof; and/or
the amount of the wetting agent is selected from 0.01-5.0% wt, preferably 0.01-4.0% wt, 0.01-3.0% wt, 0.02-2.5% wt, or 0.02-2.0% wt, more preferably 0.03-2.0% wt, more preferably 0.05-1.0% wt, still more preferably 0.1-0.5% wt.

13. The solid pharmaceutical composition according to any one of claims 6-12, wherein the disintegrant is selected from the group consisting of sodium carboxymethyl starch, dry starch, microcrystalline cellulose, hydroxyethyl methyl cellulose, sodium carboxymethyl cellulose, calcium carboxymethyl cellulose, croscarmellose sodium, low-substituted hydroxypropyl methyl cellulose or crospovidone, sodium dodecyl sulfate and magnesium dodecyl sulfate, or a mixture thereof; preferably sodium carboxymethyl starch and croscarmellose sodium, or a mixture thereof; and/or
the amount of the disintegrant is selected from 1.0-7.0% wt, preferably 1.0-6.5% wt, 1.0-6.5% wt, 1.0-6.0% wt, 1.5-5.5% wt, 1.5-5.0% wt or 1.5-4.5% wt, more preferably 2.0-4.0% wt.

14. The solid pharmaceutical composition according to any one of claims 6-13, wherein the lubricant is selected from the group consisting of magnesium stearate, colloidal silicon dioxide, talc, polyethylene glycol 4000, polyethylene glycol 6000, stearic acid, sodium stearyl fumarate and sodium dodecyl sulfate, or a mixture thereof, preferably magnesium stearate and colloidal silicon dioxide, or a mixture thereof; and/or
the amount of the lubricant is selected from 0.1-3% wt, preferably 0.2-2.5% wt, 0.3-2.0% wt or 0.4-1.5% wt, more preferably 0.5-1% wt.

15. The solid pharmaceutical composition according to any one of claims 6-14, comprising:
1-30% wt of the compound of formula **I;**
50-95% wt of microcrystalline cellulose, mannitol, lactose or pregelatinized starch, or a mixture thereof;
1.0-10% wt of hydroxypropyl methylcellulose, carboxymethyl cellulose, sodium carboxymethyl cellulose, ethyl cellulose, methyl cellulose, hydroxypropyl cellulose, L-HPC or polyvinylpyrrolidone, or a mixture thereof;
0.01-5.0% wt of sodium dodecyl sulfonate, magnesium dodecyl sulfonate, sodium tetradecyl sulfonate, sodium dodecyl sulfate, magnesium dodecyl sulfate, sodium tetradecyl sulfate, sodium hexadecyl sulfate, sodium octadecyl sulfate or sodium lauroyl sarcosine, or a mixture thereof;
1.0-7.0% wt of sodium carboxymethyl starch or croscarmellose sodium, or a mixture thereof; and
optionally, 0.1-3% wt of magnesium stearate or colloidal silicon dioxide, or a mixture thereof.

16. The solid pharmaceutical composition according to any one of claims 6-14, comprising:
2-10% wt of the compound of formula **I;**
75-95% wt of microcrystalline cellulose or mannitol, or a mixture thereof, wherein the weight ratio of microcrystalline cellulose to mannitol in the mixture is selected from 1.2:1 to 3.5:1;
1.0-6.0% wt of hydroxypropyl cellulose or polyvinylpyrrolidone, or a mixture thereof;
0.01-3.0% wt of sodium dodecyl sulfonate, magnesium dodecyl sulfonate, sodium tetradecyl sulfonate, sodium dodecyl sulfate, magnesium dodecyl sulfate, sodium tetradecyl sulfate, sodium hexadecyl sulfate, sodium octadecyl sulfate or sodium lauroyl sarcosine, or a mixture thereof;
1.0-6.0% wt of sodium carboxymethyl starch or croscarmellose sodium, or a mixture thereof; and
optionally, 0.3-2.0% wt of magnesium stearate or colloidal silicon dioxide, or a mixture thereof.

17. The solid pharmaceutical composition according to any one of claims 6-14, comprising:
3.4-4.6% wt of the compound of formula **I;**
55-60% wt of microcrystalline cellulose;
26-32% wt of mannitol;
2.0-4.0% wt of hydroxypropyl cellulose;
0.1-0.5% wt of sodium dodecyl sulfate;
2.0-4.0% wt of croscarmellose sodium; and
optionally, 0.5-1% wt of magnesium stearate.

18. The solid pharmaceutical composition according to any one of claims 6-14, comprising:
4.2% wt of the compound of formula **I;**
58.3% wt of microcrystalline cellulose;
29.4% wt of mannitol;
4.0% wt of hydroxypropyl cellulose;
0.1% wt of sodium dodecyl sulfate;
3.0% wt of croscarmellose sodium; and
optionally, 1.0% wt of magnesium stearate.

19. The solid pharmaceutical composition according to any one of claims 6-18, wherein the compound of formula I is a compound of formula II:

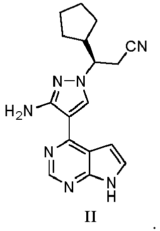

20. A method for preparing the solid pharmaceutical composition according to any one of claims 6-19, comprising:
1) mixing a diluent with a disintegrant to give a mixture for further use; mixing the compound of formula **I,** a binder, and a wetting agent with water to give a mixed solution;
2) spraying the mixed solution from step 1) to the mixture from step 1), granulating and drying through a fluidized bed, and sizing the resultant mixture to give dried granules; and
3) optionally, mixing the dried granules with a lubricant, and tableting;
wherein the mixed solution in step 1) is a suspension or a clear solution, preferably a suspension.

21. Use of the solid pharmaceutical composition according to any one of claims 6-19 in preparing a medicament for treating a disease mediated by Janus kinase, wherein preferably, the disease mediated by Janus kinase is myeloproliferative neoplasm.

22. The compound of formula **I,** the stereoisomer or the pharmaceutically acceptable salt thereof, or the pharmaceutical composition according to any one of claims 1-4, the method according to claim 5, and the use according to claim 21, wherein the myeloproliferative neoplasm is selected from polycythemia vera, thrombocythemia and myelofibrosis.

23. The compound of formula **I,** the stereoisomer or the pharmaceutically acceptable salt thereof, or the pharmaceutical composition according to any one of claims 1-4, the method according to claim 5, and the use according to claim 21, wherein the daily dose is 1 mg to 100 mg; preferably, the daily dose can be selected from the group consisting of 1 mg, 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg and 100 mg, or any range defined by endpoints of any of the foregoing values, or any value in the range.

24. The compound of formula **I,** the stereoisomer or the pharmaceutically acceptable salt thereof, or the pharmaceutical composition according to any one of claims 1-4, the method according to claim 5, and the use according to claim 21, wherein one or more doses are administered daily, optionally in a single dose; preferably, the single dosage form is administered once or twice daily.
